# EUROPEAN PATENT SPECIFICATION

(11) **EP 4 019 494 B1**
(45) Date of publication and mention of the grant of the patent: **03.12.2025**
(21) Application number: 20854442.9
(22) Date of filing: 28.07.2020
(51) Int. Cl.: C07C 263/04, C07C 265/04, C07C 265/08, C07C 265/10, C07C 265/12, C07C 265/14, C07C 233/00, C07C 269/04, C07C 271/04, C07C 68/02, C07C 69/96, C07D 251/54, C07D 263/44, C07D 295/205, B01J 19/24

(54) **METHOD FOR PRODUCING CARBONYL COMPOUND AND FLOW REACTION SYSTEM USED FOR PRODUCTION OF CARBONYL COMPOUND**
VERFAHREN ZUR HERSTELLUNG EINER CARBONYLVERBINDUNG UND DURCHFLUSSREAKTIONSSYSTEM ZUR HERSTELLUNG EINER CARBONYLVERBINDUNG
PROCÉDÉ DE PRODUCTION D'UN COMPOSÉ CARBONYLE ET SYSTÈME DE RÉACTION CONTINUE UTILISÉ POUR LA PRODUCTION D'UN COMPOSÉ CARBONYLE

(30) Priority: 22.08.2019 JP 2019152113
(43) Date of publication of application: 29.06.2022
(73) Proprietor: FUJIFILM Corporation, Tokyo 106-8620 (JP)
(72) Inventor: NISHIO, Ryo, Ashigarakami-gun, Kanagawa 258-8577 (JP); WADA, Kenji, Ashigarakami-gun, Kanagawa 258-8577 (JP)
(74) Representative: HGF
(86) International application number: PCT/JP2020/028915
(87) International publication number: WO 2021/033505

(56) References cited:
- WO-A1-2018/016376
- WO-A1-2018/016377
- WO-A1-2019/049584
- JP-A- 2005 047 875
- JP-A- 2011 026 308
- SHINICHIRO FUSE, TANABE NOBUTAKE, TAKAHASHI TAKASHI: "Continuous in situ generation and reaction of phosgene in a microflow system", CHEMICAL COMMUNICATIONS, vol. 2011, no. 47, 14 October 2011 (2011-10-14), pages 12661 - 12663, XP055580803

## Description

### BACKGROUND OF THE INVENTION

### 1. Field of the Invention

The present invention relates to a method of producing a carbonyl compound. In addition, the present invention relates to a flow type reaction system of producing a carbonyl compound.

### 2. Description of the Related Art

Phosgene is known as a reactant for introducing a carbonyl group into various compounds containing active hydrogen. For example, an isocyanate compound, a urea compound, or the like can be obtained by a reaction with a primary amine, and a carbonate compound, a chloroformate compound, or the like can be obtained by a reaction with a compound having a hydroxyl group (see, for example, WO2018/016377A, JP2011-207883A, and JP2011-006367A).

Since phosgene is extremely toxic and gaseous at room temperature, it should be handled with great care. On the other hand, triphosgene is known as a compound equivalent to the phosgene trimer. Triphosgene is solid at room temperature and is relatively safe.

In a reaction in which triphosgene is used, three molecules of phosgene are generated from triphosgene by using a tertiary amine or the like as a catalyst, and this phosgene is reacted with a reaction substrate to obtain a target carbonyl compound. As a result, even in a case where triphosgene is used, conversion to phosgene is indispensable, and safety measures such as sealing of the reaction system are required.

As a technique for dealing with this problem, WO2018/016377A describes applying a flow reactor to the above reaction. In this technique, a solution containing triphosgene and a solution containing a tertiary amine such as tributylamine and an alcohol compound are mixed and reacted in a virtually closed space called a flow reactor. According to WO2018/016377A, it is said that in a case where triphosgene is brought into contact with a tertiary amine to generate phosgene by such a reaction form, the phosgene is rapidly consumed by the alcohol compound, and as a result, it is possible to stably prevent the increase in the concentration of the highly toxic phosgene in the reaction solution.

It is noted that the tertiary amine, which acts as a catalyst for converting triphosgene into phosgene, also acts as a base for neutralizing hydrochloric acid that is generated in the reaction solution.
WO 2019/049584 describes a process for producing an organic compound using a flow reaction for a first reaction in which a raw material liquid A and raw material liquid B are introduced from separate feeding channels, mixed in a mixing unit and then reacted in a reactor unit, and a flow reactor for a second reaction in which a first reaction solution discharged from the flow reactor for the first reaction and a raw material liquid C are introduced from separate feeding channels, mixed in a mixing unit, and then reacted in a reactor unit. The liquid A is a solution in which triphosgene and/or diphosgene is dissolved, B is a nitrogen-containing organic compound as defined therein, and C is a reaction substrate as also defined therein. A product of the first reaction is phosgene.

### SUMMARY OF THE INVENTION

According to the technique described in WO2018/016377A, it is said that phosgene is generated in a closed space by using triphosgene, which is safer than phosgene, and a generated phosgene and an alcohol compound can be continuously reacted with high efficiency.

However, as a result of studies by the inventors of the present invention, it was found that in the flow type reaction specifically described in WO2018/016377A, a side reaction occurs between a tertiary amine, which catalyzes a reaction of converting triphosgene into phosgene, and phosgene. That is, it was found that the tertiary amine, which does not have active hydrogen and is conceived to hardly react with phosgene, actually reacts with phosgene to generate a by-product, which limits the improvement of the purity of the target carbonyl compound.

An object of the present invention is to provide a method of producing a carbonyl compound, which makes it possible to obtain a target carbonyl compound safely, continuously, and with high purity by using triphosgene and an active hydrogen-containing compound as starting materials. In addition, another object of the present invention is to provide a flow type reaction system suitable for carrying out the above production method.

The inventors of the present invention carried out extensive studies in consideration of the above problems. As a result of the studies, it was found that in converting triphosgene into phosgene and reacting this phosgene with an active hydrogen-containing compound to introduce a carbonyl group into the active hydrogen-containing compound, in a case where a flow type reaction using a non-aqueous organic solvent is adopted, and further, a compound having a cyclic structure is applied as a tertiary amine that is used as a catalyst for converting triphosgene into phosgene, it is possible to sufficiently efficiently convert triphosgene into phosgene, and it is possible to effectively suppress a side reaction between phosgene and the tertiary amine, whereby it is possible to dramatically increase the purity of the target reaction product. Based on these findings, further studies were repeated, and as a result, the present invention has been completed.

That is, the objects of the present invention have been achieved by the following means.
[1] A method of producing a carbonyl compound by a flow type reaction, comprising:
   introducing a triphosgene solution, a tertiary amine solution, and an active hydrogen-containing compound solution into flow channels different from each other to cause the respective solutions to flow inside the respective flow channels, joining the respective solutions that flow inside the respective flow channels simultaneously or sequentially so that a reaction between phosgene and an active hydrogen-containing compound occurs, and obtaining a carbonyl compound in a joining solution,
   in which a non-aqueous organic solvent is used as a solvent of each of the respective solutions and a compound having a cyclic structure is used as the tertiary amine; wherein
   a water content of the non-aqueous organic solvent is 1,000 ppm or less.
[3] The method of producing a carbonyl compound according to [1], in which the tertiary amine has at least one group of an alkyl group having 1 to 40 carbon atoms, a cycloalkyl group having 3 to 40 carbon atoms, an alkoxy group having 1 to 40 carbon atoms, or a polyether group having 2 to 40 carbon atoms.
[4] The method of producing a carbonyl compound according [1] or [3], in which the tertiary amine has an alkyl group having 2 to 30 carbon atoms.
[5] The method of producing a carbonyl compound according to any one of [1], [3] and [4], in which the tertiary amine has a branched alkyl group.
[6] The method of producing a carbonyl compound according to any one of [1] and [3] to [5], in which the tertiary amine has an alicyclic structure.
[7] The method of producing a carbonyl compound according to any one of [1] and [3] to [6], in which the tertiary amine has a heterocyclic ring structure having a nitrogen atom as a ring-constituting atom.
[8] The method of producing a carbonyl compound according to any one of [1] and [3] to [7], in which the tertiary amine has an oxygen atom.
[9] The method of producing a carbonyl compound according to any one of [1] and [3] to [8], in which the tertiary amine has a morpholine ring structure.
[10] The method of producing a carbonyl compound according to any one of [1] and [3] to [9], in which the tertiary amine has an aromatic heterocyclic ring structure having a nitrogen atom as a ring-constituting atom.
[11] The method of producing a carbonyl compound according to any one of [1] and [3] to [10], in which the tertiary amine has a pyridine ring structure.
[12] The method of producing a carbonyl compound according to any one [1] and [3] to [11], in which the triphosgene solution and the tertiary amine solution are joined to generate a phosgene solution, and the phosgene solution and the active hydrogen-containing compound are joined to obtain a carbonyl compound in the joining solution.
[13] The method of producing a carbonyl compound according to any one of [1] and [3] to [12], in which the active hydrogen-containing compound is at least one of a primary amine, a secondary amine, an alcohol, a thiol, a carboxylic acid, or an amino acid.
[14] The method of producing a carbonyl compound according to any one of [1] and [3] to [13], in which the active hydrogen-containing compound is a primary amine.
[15] A flow type reaction system of producing a carbonyl compound, comprising:
   a first flow channel into which a triphosgene solution is introduced; a second flow channel into which a tertiary amine solution is introduced; a third flow channel into which an active hydrogen-containing compound solution is introduced; a first joining part at which the first flow channel and the second flow channel are joined; a fourth flow channel which is connected downstream of the first joining part; a second joining part at which the fourth flow channel and the third flow channel are joined; and a reaction pipe which is connected downstream of the second joining part,
   in which a solvent of each of the solutions is a non-aqueous organic solvent, and the tertiary amine has a cyclic structure;
   wherein a water content of the non-aqueous organic solvent is 1,000 ppm or less.
[16] A flow type reaction system of producing a carbonyl compound, comprising:
   a first flow channel into which a triphosgene solution is introduced; a second flow channel into which a tertiary amine solution is introduced; a third flow channel into which an active hydrogen-containing compound solution is introduced; a joining part at which the first flow channel, the second flow channel, and the third flow channel are joined; and a reaction pipe which is connected downstream of the joining part,
   in which a solvent of each of the solutions is a non-aqueous organic solvent, and the tertiary amine has a cyclic structure;
   wherein a water content of the non-aqueous organic solvent is 1,000 ppm or less.

In the present specification, numerical ranges expressed using "to" include numerical values before and after the "to" as the lower limit value and the upper limit value.

In a case where an intra-pipe cross-sectional size (an equivalent diameter) of a flow channel, a joining part, a mixer, or the like is described in the present specification, the above size refers to a size excluding a connecting portion between flow channels, a connecting portion between a flow channel and a joining part, or a connecting portion between a flow channel and a mixer. That is, the size of each of the above connecting portions is appropriately adjusted by using a connecting tube or the like so that a fluid flows through the connecting portion from the upstream to the downstream.

In the present specification, in a case where the number of carbon atoms of a certain group is specified, this number of carbon atoms means the number of carbon atoms of the entire group. That is, in a case where this group has a form of further having a substituent, it means the total number of carbon atoms, to which the number of carbon atoms of this substituent is included.

According to the method of producing a carbonyl compound according to an aspect of the present invention, a target carbonyl compound can be obtained safely, continuously, and with high purity. Further, in the flow type reaction system according to an aspect of the present invention, a target carbonyl compound can be obtained safely, continuously, and with high purity by carrying out the above-described production method using the flow type reaction system.

### BRIEF DESCRIPTION OF THE DRAWINGS

Fig. 1 is an illustrative view illustrating an outline of one embodiment of a flow type reaction system according to the embodiment of the present invention.
Fig. 2 is an illustrative view illustrating an outline of another embodiment of a flow type reaction system according to the embodiment of the present invention.

### DESCRIPTION OF THE PREFERRED EMBODIMENTS

### [Production of carbonyl compound by flow type reaction]

In a method of producing a carbonyl compound according to the embodiment of the present invention (hereinafter, also referred to as a "production method according to the embodiment of the present invention"), a flow type reaction is adopted. In carrying out this flow type reaction, a triphosgene solution obtained by dissolving triphosgene in a non-aqueous organic solvent, a tertiary amine solution obtained by dissolving a tertiary amine in a non-aqueous organic solvent, and an active hydrogen-containing compound solution obtained by dissolving an active hydrogen-containing compound in a non-aqueous organic solvent are prepared. In the present invention, the "non-aqueous organic solvent" means an organic solvent having a water content of 2,000 ppm or less. The water content of the "non-aqueous organic solvent" is preferably 1,000 ppm or less. In the present invention, "ppm" is based on mass.

In addition, in the present invention, the "active hydrogen" means a hydrogen atom bonded to a nitrogen atom, an oxygen atom, or a sulfur atom.

The above respective solutions are introduced into flow channels different from each other and flow inside the respective flow channels. In a case where the respective solutions that flow inside the respective flow channels are joined simultaneously or sequentially, the tertiary amine acts as a catalyst for converting triphosgene into phosgene, whereby phosgene is generated. Then, this phosgene reacts with an active hydrogen-containing compound, and a carbonyl group is introduced into the active hydrogen-containing compound. That is, a carbonyl compound is obtained in the joining solution while the joining solution flows downstream.

In the present specification, the terms "upstream" and "downstream" are used with respect to the direction in which a liquid flows, and a side where a liquid is introduced (a side where a liquid flows in) is upstream, and a side where a liquid flows out is downstream.

In the present invention, a compound having a cyclic structure is used as the tertiary amine. As will be described in detail later, due to having a cyclic structure, the tertiary amine realizes highly efficient conversion as a catalyst for converting triphosgene into phosgene, and a side reaction between the tertiary amine and phosgene hardly occurs. In addition, it can effectively function as a highly soluble neutralizing agent in a solvent with respect to hydrochloric acid that is generated in the reaction solution.

In a case where the above respective solutions are joined sequentially, the order of joining is not particularly limited. Preferably, a preferred configuration is a configuration in which a triphosgene solution and a tertiary amine solution are joined in advance to convert triphosgene into phosgene, and then this phosgene solution and an active hydrogen-containing compound solution are joined to introduce a carbonyl group into the active hydrogen-containing compound. Even in such a configuration of joining, a side reaction hardly occurs between the phosgene and the tertiary amine in the present invention in which a compound having a cyclic structure is used as the tertiary amine. After sufficient phosgene is generated, the generated phosgene can be reacted with an active hydrogen-containing compound, which is a reaction substrate, and a desired carbonyl compound having high purity can be obtained.

In the flow type reaction according to the embodiment of the present invention, it is preferable that the temperature in the reaction flow channel is set to be at least lower than a boiling point of a solvent of which the boiling point is lowest among solvents that are used in the reaction. This makes it possible to carry out the reaction more reliably in the liquid phase state. It is noted that in a case where one kind of solvent is used, the above-described "lower than a boiling point of a solvent of which the boiling point is lowest" is lower than the boiling point of this one kind of solvent.

One embodiment of the flow type reaction system that is used in the present invention will be described with reference to the drawings. It is be noted that each drawing is an illustrative view for facilitating the understanding of the present invention, and the magnitude of the size, the relative magnitude relationship, or the like of each member may be changed for the convenience of description, and it does not indicate the actual magnitude relationship as it is. Further, matters other than those specified in the present invention are not limited to the outer shape and the shape illustrated in these drawings.

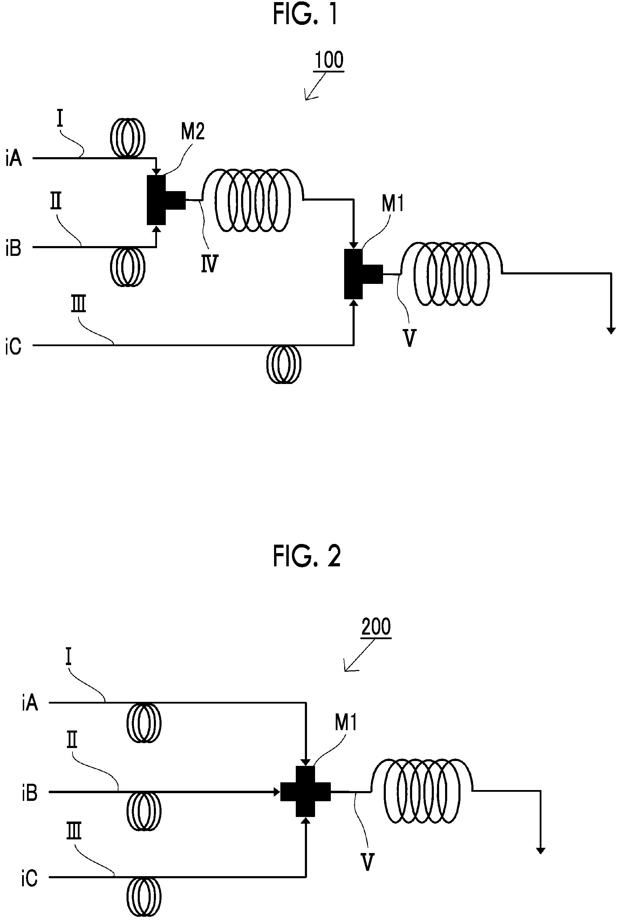

Fig. 1 is a schematic view illustrating an example of a flow type reaction system that is used in the production method according to the embodiment of the present invention. A flow type reaction system (100) illustrated in Fig. 1 has a flow channel (I) having an introduction port (iA) into which a triphosgene solution is introduced, a flow channel (II) having an introduction port (iB) into which a tertiary amine solution is introduced, and a flow channel (III) having an introduction port (iC) into which an active hydrogen-containing compound solution is introduced.

The flow channel (I) and the flow channel (II) are joined at the joining part (M2), and a flow channel (IV) is connected to the downstream end portion of a joining part (M2). This flow channel (IV) and the flow channel (III) are joined at the joining part (M1), and a reaction pipe (V) is connected to the downstream end portion of a joining part (M1).

In the inside of the joining pipe (IV), the tertiary amine acts on triphosgene to generate a phosgene solution, this phosgene reacts with the active hydrogen-containing compound in the reaction pipe (V) to generate a carbonyl compound.

Liquid feeding pumps (not illustrated in the drawing) such as syringe pumps are usually connected to the introduction ports (iA), (iB), and (iC), respectively, and in a case where these pumps are operated, it is possible to allow a triphosgene solution, a tertiary amine solution, and an active hydrogen-containing compound solution to flow inside flow channels, respectively, at a desired flow speed.

Each configuration of the embodiment illustrated in Fig. 1 will be described in more detail.

### <Flow channel (I)>

The flow channel (I) is a flow channel in which the triphosgene solution introduced from the introduction port (iA) is supplied to the joining part (M2).

The flow channel (I) is preferably set to have an equivalent diameter of 0.2 to 50 mm. In a case where the equivalent diameter of the flow channel (I) is set to 0.2 mm or more, it is possible to suppress an increase in pressure during liquid feeding, and it is possible to suppress the clogging of the flow channel even in a case where an insoluble matter is generated. In addition, in a case where the equivalent diameter of the flow channel (I) is set to 50 mm or less, it is possible to suitably control the liquid temperature at the time of being introduced into joining part (M2). The equivalent diameter of the flow channel (I) is more preferably 0.5 to 30 mm and still more preferably 1 to 20 mm.

The "equivalent diameter" is a term used in the field of mechanical engineering. In a case of assuming a circular pipe that is equivalent to a pipe or flow channel having any intra-pipe cross-sectional shape, a diameter of the intra-pipe cross-section of the equivalent circular pipe is referred to as the equivalent diameter. The equivalent diameter (deq) is defined by using A: an intra-pipe cross-sectional area of a pipe, and p: a wetted perimeter (inner circumference) of the pipe, as deq = 4A/p. In a case of being applied to a circular pipe, this equivalent diameter corresponds to the diameter of the intra-pipe cross section of the circular pipe. The equivalent diameter is used to estimate the flow or the heat transfer characteristics of a pipe based on the data of the equivalent circular pipe and represents the spatial scale (the representative length) of the phenomenon. In a case of a square pipe in which the intra-pipe cross section has a side of a, the equivalent diameter is deq = 4a²/4a = a, in a case of an equilateral triangle pipe in which the intra-pipe cross section has a side of a, the equivalent diameter is deq = a/3^{1/2}, and in a case of a flow between flat plates parallel to the flow channel having a height of h, the equivalent diameter is deq = 2h (see, for example, "Mechanical Engineering Dictionary" edited by The Japan Society of Mechanical Engineers, 1997, Maruzen Publishing Co., Ltd.).

The length of the flow channel (I) is not particularly limited, and for example, it can be constituted of a tube having a length of about 10 cm to 15 m (preferably 30 cm to 10 m).

The material of the tube is not particularly limited, and examples thereof include a perfluoroalkoxy alkane (PFA), Teflon (registered trade name), an aromatic polyether ketone-based resin, stainless steel, copper or a copper alloy, nickel or a nickel alloy, titanium or a titanium alloy, quartz glass, and lime soda glass. From the viewpoint of flexibility and chemical resistance, the material of the tube is preferably PFA, Teflon (registered trade name), stainless steel, a nickel alloy, or titanium is preferable.

The flow speed for introducing the triphosgene solution from the introduction port (iA) is not particularly limited, and it can be appropriately set depending on the intended purpose in consideration of the equivalent diameter of the flow channel, the concentration of the triphosgene solution, the concentration of the tertiary amine solution, the introduction flow rate of the tertiary amine solution, the concentration of the active hydrogen-containing compound solution, the introduction flow rate of the active hydrogen-containing compound solution, and the like. For example, 0.1 to 5,000 mL/minutes (min) is preferable, 0.5 to 3,000 mL/min is more preferable, and 1 to 3,000 mL/min is still more preferable.

### - Triphosgene solution -

The triphosgene solution that is introduced into the flow channel (I) is a solution obtained by dissolving triphosgene in a non-aqueous organic solvent. Examples of this non-aqueous organic solvent include a halogen-containing solvent, an ether solvent having a linear, branched, or cyclic structure, and a hydrocarbon solvent.

Examples of the halogen-containing solvent include methylene chloride, chloroform, dichloroethane, carbon tetrachloride, chlorobenzene, and o-dichlorobenzene.

Examples of the ether solvent include tetrahydrofuran, dioxane, methyl tertiary butyl ether, cyclopentyl methyl ether, ethylene glycol dibutyl ether, diethylene glycol dimethyl ether, diethylene glycol diethyl ether, diethylene glycol dibutyl ether, and derivatives thereof.

Examples of the hydrocarbon solvent include hexane, heptane, octane, cyclohexane, methyl cyclohexane, benzene, toluene, xylene, mesitylene, decalin, tetralin, and derivatives thereof.

In addition, the following can be used as the above non-aqueous organic solvent; a ketone-based solvent such as acetone, methyl ethyl ketone, diisobutyl ketone, cyclohexanone, or methyl isobutyl ketone, a nitrile-based solvent such as acetonitrile, a lactone-based solvent such as γ-butyrolactone, an ester-based solvent such as ethyl acetate or butyl acetate, and an amide-based solvent such as dimethyl acetamide or dimethyl formamide.

The above non-aqueous organic solvent may be used alone, or two or more kinds thereof may be used in a state of being mixed.

Among them, at least one of methylene chloride, chloroform, chlorobenzene, o-dichlorobenzene, tetrahydrofuran, dioxane, toluene, xylene, mesitylene, cyclohexanone, methyl ethyl ketone, or acetonitrile is preferably used, at least one of methylene chloride, chlorobenzene, o-dichlorobenzene, tetrahydrofuran, toluene, xylene, mesitylene, or acetonitrile is more preferably used, and at least one of methylene chloride, toluene, mesitylene, chlorobenzene, or acetonitrile is still more preferably used.

In a case where the water content in the organic solvent is large, the organic solvent can be made to be a non-aqueous organic solvent by appropriately bringing the organic solvent into contact with a commercially available dehydrating agent (a molecular sieve, anhydrous sodium sulfate, anhydrous magnesium sulfate, or the like) to remove the water content.

The content of the triphosgene in the triphosgene solution is not particularly limited, and it is appropriately adjusted in consideration of the introduction flow rate of the triphosgene solution, the concentration of the tertiary amine solution, the introduction flow rate of the tertiary amine solution, the concentration of the active hydrogen-containing compound solution, the introduction flow rate of the active hydrogen-containing compound solution, and the like. The content of triphosgene in the triphosgene solution can be, for example, 0.01 to 10 M (mol/liter), and it is preferably 0.03 to 3 M and more preferably 0.05 to 1 M.

The temperature of the flow channel (I) is preferably set to be lower than the boiling point of the solvent used to prepare the triphosgene solution. For example, it can be set to -60°C to 80°C, and it is preferably -20°C to 30°C and more preferably -10°C to 20°C.

### <Flow channel (II)>

The flow channel (II) is a flow channel in which the tertiary amine solution introduced from the introduction port (iB) is supplied to the joining part (M2). The flow channel (II) is preferably set to have an equivalent diameter of 0.1 to 50 mm. In a case where the equivalent diameter of the flow channel (II) is set to 0.1 mm or more, it is possible to suppress an increase in pressure during liquid feeding, and it is possible to suppress the clogging of the flow channel even in a case where an insoluble matter is generated. In addition, in a case where the equivalent diameter of the flow channel (II) is set to 50 mm or less, it is possible to suitably control the liquid temperature at the time of being introduced into joining part (M2). The equivalent diameter of the flow channel (II) is more preferably 0.5 to 30 mm and still more preferably 1 to 20 mm.

The length of the flow channel (II) is not particularly limited, and for example, it can be constituted of a tube having a length of about 10 cm to 15 m (preferably 30 cm to 10 m).

The material of the tube is not particularly limited, and the tube of the material exemplified in the above flow channel (I) can be used.

The flow speed for introducing the tertiary amine solution from the introduction port (iB) is not particularly limited, and it can be appropriately set depending on the intended purpose in consideration of the equivalent diameter of the flow channel, the concentration of the tertiary amine solution, the concentration of the triphosgene solution, the introduction flow rate of the triphosgene solution, the concentration of the active hydrogen-containing solution, the introduction flow rate of the active hydrogen-containing compound solution, and the like. For example, 0.1 to 5,000 mL/minutes (min) is preferable, 0.5 to 3,000 mL/min is more preferable, and 1 to 3,000 mL/min is still more preferable. In a case where the introduction flow rate of the tertiary amine solution is set within the above range, side reactions can be suppressed and the purity can be further improved.

In addition, the relationship between the flow speed rB for introducing the tertiary amine solution from the introduction port (iB) and the flow speed rA for introducing the triphosgene solution from the introduction port (iA) is not particularly limited, and the flow speed therefor can be appropriately set in consideration of the concentrations of the respective solutions. For example, the relationship therebetween can be set to [flow speed rA]/[flow speed rB] = 10/1 to 1/10, and it is preferably [flow speed rA]/[flow speed rB] = 5/1 to 1/5. It is noted that in the present specification, the unit of the flow speed is mL/min.

### - Tertiary amine solution -

The tertiary amine solution that is allowed to flow inside the flow channel (II) is a solution obtained by dissolving a tertiary amine having a specific structure described later in a non-aqueous organic solvent. As the non-aqueous organic solvent, those exemplified as the non-aqueous organic solvent of the above-described triphosgene solution can be preferably used. The tertiary amine solution and the triphosgene solution may use the same solvent, or the kinds of solvents thereof may be different from each other. In a case where the kinds of solvents thereof are different from each other, it is preferable to use solvents that are compatible with each other (solvents that do not phase-separate in a case of being mixed).

### (Tertiary amine)

In the present invention, the term "tertiary amine" is used in a broader sense than usual. That is, all amines in which a hydrogen atom is not bonded to a nitrogen atom (amines in which all three bonding sites of the nitrogen atom are bonded to an atom other than the hydrogen atom) are included in the "tertiary amine". For example, a compound having an aromatic ring (for example, a pyridine ring, a pyridazine ring, a pyrimidine ring, a pyrazine ring, a 2H-pyrrole ring, an oxazole ring, an isoxazole ring, or a thiazole ring, an isothiazole ring) which has a nitrogen atom as a ring-constituting atom and in which the nitrogen atom which is a ring-constituting atom does not have an active hydrogen atom is also the tertiary amine in the present invention. In addition, a configuration in which in a compound having a pyrrole ring, a pyrazole ring, an imidazole ring, or the like, which is an aromatic ring, a hydrogen atom possessed by the nitrogen atom which is a ring-constituting atom is substituted with another substituent is also the tertiary amine in the present invention.

The tertiary amine of the tertiary amine solution preferably does not have active hydrogen even in the structural part other than the amino group.

In the present invention, the tertiary amine in the tertiary amine solution has a cyclic structure. The cyclic structure may be an aromatic ring or an alicyclic ring.

According to the studies by the inventors of the present invention, it was revealed that even in a case of a tertiary amine containing no active hydrogen, a side reaction between the tertiary amine and phosgene occurs, which limits the improvement of the yield or purity of the target carbonyl compound. An example of this side reaction is shown below. In the side reaction schemes below, "Ph" is phenyl. In addition, a by-product generated via a quaternary salt as in the scheme below is also referred to as a by-product via the quaternary salt.

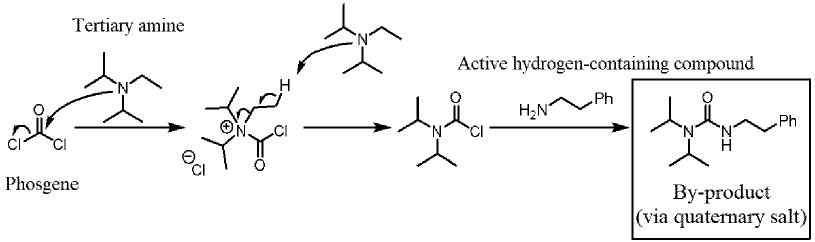

The inventors of the present invention could sufficiently suppress the above-described side reaction and have succeeded in obtaining a target carbonyl compound with high purity in a case where a compound having a cyclic structure was applied as the tertiary amine. That is, as specified in the present invention, in a case where the tertiary amine has a cyclic structure, it is possible to realize, at a high level, the achievement of both the higher efficiency of converting triphosgene into phosgene and the suppression of the side reactions between the tertiary amine and the phosgene. The reason for this is not clear. However, regarding the efficiency of conversion of triphosgene into phosgene, the following is conceived to be one of the causes; in a case where the substituent of the tertiary amine has a cyclic structure, a suitable steric hindrance is obtained, and thus the reversible action of the tertiary amine on triphosgene becomes highly efficient (not only the addition reaction but also the elimination reaction becomes highly efficient) as compared with a case where the substituent of the tertiary amine has a linear structure. In addition, regarding the suppression of the side reaction between the tertiary amine and the phosgene, the following is conceived to be one of the causes; the substituent contained in the tertiary amine is difficult to be eliminated due to suitable steric hindrance. Further, the tertiary amine having a cyclic structure exhibits high solvent solubility, and thus even in a case where it forms a salt together with hydrochloric acid, the salt is difficult to be precipitated and the clogging of the flow channel hardly occurs.

The cyclic structure of the tertiary amine is preferably a 5-membered ring or a 6-membered ring. In addition, this cyclic structure may be a fused-ring structure (a structure in which a ring selected from a 5-membered ring and a 6-membered ring is fused). In a case of a fused-ring structure, the number of rings that constitute the fused ring is preferably 2 or 3 and more preferably 2. The cyclic structure of the tertiary amine is more preferably monocyclic.

The cyclic structure of the tertiary amine may be an alicyclic structure or an aromatic ring structure. In addition, the nitrogen atom that constitutes the tertiary amine may be a ring-constituting atom or may not be a ring-constituting atom (the tertiary amine may be contained in the substituent contained in the ring). The cyclic structure of the tertiary amine is more preferably a heterocyclic ring structure having a nitrogen atom as a ring-constituting atom. This heterocyclic ring structure may be an alicyclic ring or an aromatic ring. In addition, the above tertiary amine preferably has an oxygen atom or preferably has an oxygen atom as a ring-constituting atom.

Specific examples of the cyclic structure of the tertiary amine include a morpholine ring, a piperazine ring, a piperidine ring, a 2-pyrroline ring, a pyrrolidine ring, a 2-imidazoline ring, an imidazolidine ring, a pyrazoline ring, a pyrazolidine ring, a pyridine ring, a pyridazine ring, a pyrimidine ring, a pyrazine ring, a 2H-pyrrole ring, an oxazole ring, an isoxazole ring, a thiazole ring, an isothiazole ring, a pyrrole ring, a pyrazole ring, an imidazole ring, an indole ring, an isoindole ring, a 1H-indole ring, a quinoline ring, an isoquinoline ring, a cinnoline ring, a phthalazine ring, a quinazoline ring, a quinoxaline ring, a 1,8-naphthyridine ring, a purine ring, a pteridine ring, an indolizine ring, a carbazole ring, an acridine ring, a phenazine ring, a phenanthridine ring, a 1,10-phenanthroline ring, a phenoxazine ring, and a quinuclidine ring.

The cyclic structure that can be included in the tertiary amine is preferably a ring selected from a morpholine ring, a pyridine ring, a piperazine ring, a piperidine ring, a pyrrolidine ring, an imidazole ring, a quinoline ring, and a thiomorpholine ring, and it is more preferably a ring selected from a morpholine ring and a pyridine ring.

The tertiary amine of the tertiary amine solution preferably has, in the molecule thereof, at least one of an alkyl group having 1 to 40 carbon atoms, a cycloalkyl group having 3 to 40 carbon atoms, an alkoxy group having 1 to 40 carbon atoms, or a polyether group having 2 to 40 carbon atoms, and among the above, it more preferably has an alkyl group having 2 to 30 carbon atoms. In addition, the above tertiary amine preferably has a form of having a branched alkyl group. The number of carbon atoms in this branched alkyl group is preferably 3 to 30, more preferably 3 to 20, and still more preferably 3 to 12.

The molecular weight of the tertiary amine that is used in the present invention is preferably 100 to 700 and more preferably 100 to 450.

Specific examples of the above tertiary amine are shown below.

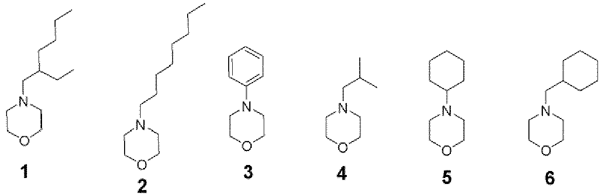

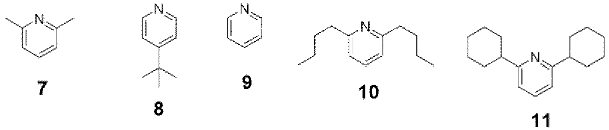

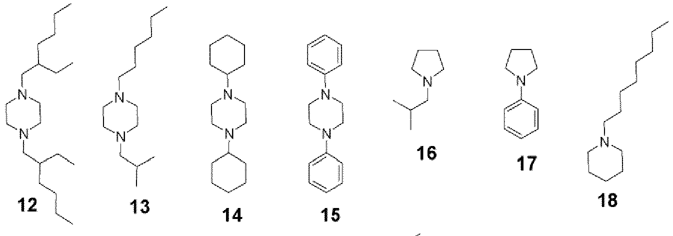

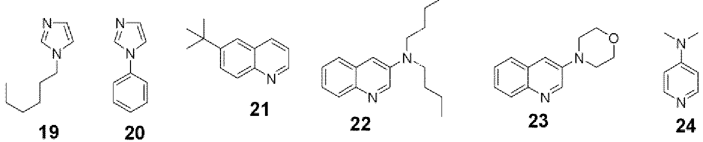

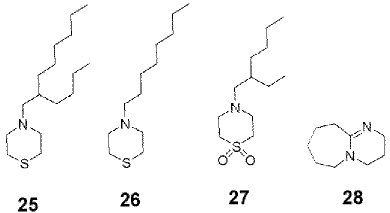

Among the above-exemplified tertiary amines, it is preferable to use at least one of the tertiary amines of Nos. 1 to 27, it is more preferable to use at least one of the tertiary amines of Nos. 1 to 8, 10, 12 to 15, 18, 23, 25, or 26, it is still more preferable to use at least one of the tertiary amines of Nos. 1, 2, 4 to 8, 10, 12, 14, or 18, it is even still more preferable to use at least one of the tertiary amines of No. 1, 2, 4, 6, 7, 8, or 10, and it is even further still more preferable to use at least one of the tertiary amines of No. 1 or 7.

The content of the tertiary amine in the tertiary amine solution is not particularly limited, and it is appropriately adjusted in consideration of the introduction flow rate of the tertiary amine solution, the concentration of the triphosgene solution, the introduction flow rate of the triphosgene solution, the concentration of the active hydrogen-containing compound solution, the introduction flow rate of the active hydrogen-containing compound solution, and the like. The content of the tertiary amine in the tertiary amine solution can be, for example, 0.03 to 10 M (mol/liter), and it is preferably 0.05 to 7 M and more preferably 0.1 to 5 M.

The temperature of the flow channel (II) is preferably set to be lower than the boiling point of the solvent used to prepare the tertiary amine solution. For example, it can be set to -60°C to 80°C, and it is preferably -20°C to 30°C and still more preferably -10°C to 20°C.

### <Joining part M2>

In the embodiment illustrated in Fig. 1, the flow channel (I) through which the triphosgene solution flows and the flow channel (II) through which the tertiary amine solution flows are joined at the joining part (M2), and the triphosgene solution is converted into a phosgene solution by the catalytic action of the tertiary amine while the joining solution flows downstream through the flow channel (IV).

The connection method between the flow channel (I) and the flow channel (II) (the form of the joining part (M2)) is not particularly limited, and for example, a T-shaped or Y-shaped connector can be used. The material of this connector is preferably, for example, a perfluoroalkoxy alkane (PFA), Teflon (registered trade name), an aromatic polyether ketone-based resin, stainless steel, copper or a copper alloy, nickel or a nickel alloy, titanium or a titanium alloy, quartz glass, or lime soda glass. As the commercially available product of the above connector, the following can be used; Microglass Reactor manufactured by Microglass; Cytos manufactured by CPC Systems Ltd.; YM-1 and YM-2 type mixers manufactured by Yamatake Co., Ltd.; a mixing tee and a tee (T-shaped connectors) manufactured by SHIMADZU GLC Ltd.; a mixing tee and a tee (T-shaped connectors) manufactured by GL Sciences Inc.; a mixing tee and a tee (T-shaped connectors) manufactured by Upchurch Scientific Inc.;; a mixing tee and a tee (T-shaped connectors) manufactured by Upchurch Scientific Inc.; a mixing tee and a tee (T-shaped connectors) manufactured by Valco Instruments Co. Inc.; a T-shaped connector manufactured by Swagelok Company; and a SUS T-type mixer manufactured by IDEX CORPORATION. Any one of these can be used in the present invention

The molar ratio of the triphosgene to the tertiary amine in the joining solution immediately after being joined at the joining part (M2) can be set to, for example, [triphosgene]:[tertiary amine] = 1:3 to 12, and it is preferably [triphosgene]: [tertiary amine] = 1:6 to 8.

### <Flow channel (IV)>

The flow channel (IV) is a flow channel that supplies a joining solution of the triphosgene solution and the tertiary amine solution, which are joined at the joining part (M2), to the joining part (M1) while generating phosgene in the joining solution. The flow channel (IV) is preferably set to have an equivalent diameter of 0.1 to 50 mm. In a case where the equivalent diameter of the flow channel (IV) is set to 0.1 mm or more, it is possible to suppress an increase in pressure during liquid feeding, and it is possible to suppress the clogging of the flow channel even in a case where an insoluble matter is generated. In addition, in a case where the equivalent diameter of the flow channel (IV) is set to 50 mm or less, it is possible to suitably control the liquid temperature inside the flow channel. The equivalent diameter of the flow channel (IV) is more preferably 0.5 to 30 mm and still more preferably 1 to 20 mm.

The length of the flow channel (IV) is not particularly limited, and for example, it can be constituted of a tube having a length of about 10 cm to 15 m (preferably 30 cm to 10 m).

The material of the tube is not particularly limited, and the tube of the material exemplified in the above flow channel (I) can be used.

The reaction time for generating phosgene from triphosgene using a tertiary amine as the conversion catalyst can be appropriately adjusted by setting the equivalent diameter and the length of the flow channel (IV), the flow rate of the liquid feeding pump, and the like. For example, the time during which the joining solution of the triphosgene solution and the tertiary amine solution flows inside the flow channel (IV) can be set to 3 to 600 seconds, and it is preferably 5 to 200 seconds.

### <Flow channel (III)>

The flow channel (III) is a flow channel in which the active hydrogen-containing compound solution introduced from the introduction port (iC) is supplied to the joining part (M1). The flow channel (III) is preferably set to have an equivalent diameter of 0.1 to 50 mm. In a case where the equivalent diameter of the flow channel (III) is set to 0.1 mm or more, it is possible to suppress an increase in pressure during liquid feeding, and it is possible to suppress the clogging of the flow channel even in a case where an insoluble matter is generated. In addition, in a case where the equivalent diameter of the flow channel (III) is set to 50 mm or less, it is possible to suitably control the liquid temperature at the time of being introduced into joining part (M1). The equivalent diameter of the flow channel (III) is more preferably 0.5 to 30 mm and still more preferably 1 to 20 mm.

The length of the flow channel (III) is not particularly limited, and for example, it can be constituted of a tube having a length of about 10 cm to 15 m (preferably 30 cm to 10 m).

The material of the tube is not particularly limited, and the tube of the material exemplified in the above flow channel (I) can be used.

The flow speed for introducing the active hydrogen-containing compound solution from the introduction port (iC) is not particularly limited, and it can be appropriately set depending on the intended purpose in consideration of the equivalent diameter of the flow channel, the concentration of the triphosgene solution, the concentration of the active hydrogen-containing compound solution, the introduction flow rate of the triphosgene solution, and the like. For example, 0.1 to 5,000 mL/minutes (min) is preferable, 0.5 to 3,000 mL/min is more preferable, and 1 to 3,000 mL/min is still more preferable.

In addition, the relationship between the flow speed rC for introducing the active hydrogen-containing compound solution from the introduction port (iC) and the flow speed rD of the joining solution that flows inside the flow channel (IV) is not particularly limited, and the flow speed therefor can be appropriately set in consideration of the concentrations of the respective solutions. For example, the relationship therebetween can be set to [flow speed rC]/[flow speed rD] = 10/1 to 1/10, and it is preferably [flow speed rC]/[flow speed rD] = 5/1 to 1/5. It is noted that in the present specification, the unit of the flow speed is mL/min.

### - Active hydrogen-containing compound solution -

The active hydrogen-containing compound solution that is allowed to flow inside the flow channel (III) is a solution obtained by dissolving an active hydrogen-containing compound in a non-aqueous organic solvent. As the non-aqueous organic solvent, those exemplified as the non-aqueous organic solvent of the above-described triphosgene solution can be preferably used. The active hydrogen-containing compound solution, and the triphosgene solution or the tertiary amine solution may use the same solvent, or the kinds of solvents thereof may be different from each other. In a case where the kinds of solvents thereof are different from each other, it is preferable to use solvents that are compatible with each other (solvents that do not phase-separate in a case of being mixed).

### (Active hydrogen-containing compound)

An active hydrogen-containing compound in the active hydrogen-containing compound solution is not particularly limited, and for example, a compound having at least one group selected from -OH, -COOH, -NH₂, -NHR (R is a substituent), or -SH can be widely used. The active hydrogen-containing compound is, for example, at least one of a primary amine, a secondary amine, an alcohol, a thiol, a carboxylic acid, or an amino acid.

The reaction itself for introducing a carbonyl group by reacting this active hydrogen-containing group with phosgene is known, and the reaction conditions and the like are appropriately set depending on the target reaction. An isocyanate compound, a carbamoyl chloride compound, a urea compound, or the like can be obtained by reacting a compound having -NH₂ with phosgene as an example of the above reaction. In addition, a carbonate compound, a chloroformate compound, or the like can be obtained by reacting a compound having -OH with phosgene. In addition, an acid chloride compound can be obtained by reacting a compound having -COOH with phosgene. Further, an amino acid anhydride can be obtained by reacting an amino acid with phosgene.

Among the above, the active hydrogen-containing compound is preferably a primary amine, a secondary amine, an alcohol, or an amino acid, and it is more preferably a primary amine.

The active hydrogen-containing compound, which is a reaction substrate, preferably has a molecular weight of 40 to 1,000 and more preferably 60 to 500.

The content of the active hydrogen-containing compound in the active hydrogen-containing compound solution is not particularly limited, and it is appropriately adjusted in consideration of the introduction flow rate of the active hydrogen-containing compound solution, the concentration of the triphosgene solution, the introduction flow rate of the triphosgene solution, and the like. The content of the active hydrogen-containing compound in the active hydrogen-containing compound solution can be set to, for example, 0.02 to 10 M (mol/liter) and it is preferably 0.05 to 3 M and more preferably 0.07 to 1 M.

The temperature of the flow channel (III) is preferably set to be lower than the boiling point of the solvent used to prepare the active hydrogen-containing compound solution. For example, it can be set to -60°C to 80°C, and it is preferably -20°C to 30°C and still more preferably -10°C to 20°C.

### <Joining part (M1)>

The joining solution (the solution containing the phosgene and the tertiary amine) that flows inside the flow channel (IV) and the active hydrogen-containing compound solution that flows inside the flow channel (III) are joined at the joining part (M1). The joining part (M1) is not particularly limited as long as it has a role of a mixer, can join the flow channel (IV) and the flow channel (III) into one flow channel, and can send the joined solution to the reaction pipe (V) that is connected to the downstream end portion of the joining part (M1). For example, a T-shaped or Y-shaped connector can be used.

In the embodiment of Fig. 1, a T-shaped connector is used as the joining part (M1). The equivalent diameter of the flow channel in the joining part (M1) is preferably 0.1 to 30 mm from the viewpoint of further improving the mixing performance.

The material of the joining part (M1) is not particularly limited, and for example, the same material as that described in the joining part (M2) can be used.

### <Reaction pipe (V)>

The joining solution joined at the joining part (M1) flows inside the reaction pipe (V), which is a reaction flow channel, and while they flow downstream inside the reaction pipe (V), phosgene reacts with an active hydrogen-containing compound in the presence of the tertiary amine. In the present specification, the reaction pipe (V) may be referred to as a flow channel (V).

The form of the reaction pipe (V) is not particularly limited, and a tube is generally used. The preferred material of the reaction pipe (V) is the same as the preferred material of the flow channel (I) described above. In addition, the reaction time can be adjusted by setting the equivalent diameter and the length of the reaction pipe (V), the flow rate of the liquid feeding pump, and the like. Generally, the equivalent diameter of the reaction pipe (V) is preferably 0.1 to 50 mm, more preferably 0.2 to 20 mm, still more preferably 0.4 to 15 mm, even still more preferably 0.7 to 12 mm, and even further still more preferably 1 to 10 mm. In addition, the length of the reaction pipe (V) is preferably 0.5 to 50 m and more preferably 1 to 30 m.

At the time of feeding liquids of raw materials, the molar ratio between triphosgene, the active hydrogen-containing compound, and the tertiary amine is appropriately set depending on the target reaction. For example, it can be set to [triphosgene]:[active hydrogen-containing compound]: [tertiary amine] = 0.1 to 2:1:0.6 to 12, and it is preferably [triphosgene]:[active hydrogen-containing compound]:[tertiary amine] = 0.35 to 1.5:1:2 to 9.

The temperature of the reaction pipe (V) is preferably set to be lower than a boiling point of a solvent of which the boiling point is lowest among solvents in the reaction solution that flows inside the reaction pipe (V) (in a case where the solvent is one kind, it is preferably lower than the boiling point of this one kind of solvent). For example, the temperature of the reaction pipe (V) can be set to -60°C to 80°C, and it is preferably -20°C to 30°C and still more preferably -10°C to 20°C.

Another embodiment of the flow type reaction system for carrying out the production method of the present invention will be described with reference to Fig. 2.

The flow type reaction system (200) illustrated in Fig. 2 adopts a configuration in which the flow channel (I) through which the triphosgene solution flows, the flow channel (II) through which the tertiary amine solution flows, and the flow channel (III) through which the active hydrogen-containing compound solution flows are simultaneously mixed at the joining part (M1). The constitution other than the above is the same as that described in the embodiment of Fig. 1.

### <Joining part M1>

In the embodiment of Fig. 2, the joining part (M1) is not particularly limited as long as it has a role of a mixer, can join the flow channel (I), the flow channel (II), and the flow channel (III) into one flow channel, and can send the joined solution to the reaction pipe (V) that is connected to the downstream end portion of the joining part (M1).

The equivalent diameter of the flow channel in the joining part (M1) is preferably 0.2 to 50 mm from the viewpoint of further improving the mixing performance.

The material of the joining part (M1) is not particularly limited, and a material consisting of, for example, a perfluoroalkoxy alkane (PFA), Teflon (registered trade name), an aromatic polyether ketone-based resin, stainless steel, copper or a copper alloy, nickel or a nickel alloy, titanium or a titanium alloy, quartz glass, lime soda glass, or the like can be used.

The joining part (M1) can be constituted with a cross-shaped connector. A commercially available product can be widely used as this cross-shaped connector, and the following can be used as the commercially available product, for example; a cross-shaped connector manufactured by Upchurch Scientific Inc.; a union cross manufactured by Swagelok Company; a 4-way joint manufactured by TOKYO RIKAKIKAI Co, Ltd., a SUS cross mixer manufactured by IDEX CORPORATION, or the like.

In the embodiments of Figs. 1 and 2, the retention time (the reaction time) of the reaction solution (joining solution) in the reaction pipe (V) is preferably set to 2 seconds or more, more preferably 3 to 600 seconds, and still more preferably 5 to 200 seconds. In a case where the reaction time is shortened to some extent, side reactions can be suppressed more effectively.

According to the production method according to the embodiment of the present invention, a tertiary amine having a cyclic structure is used as the tertiary amine that is used as a catalyst for converting triphosgene into phosgene. This makes it possible to dramatically reduce by-products that are generated by the reaction between the tertiary amine and the phosgene while sufficiently increasing the efficiency of converting triphosgene into phosgene and makes it possible to obtain a desired carbonyl with high purity. In addition, the solubility in the non-aqueous organic solvent is excellent, and even in a case where an ammonium salt is formed in association with hydrochloric acid, the salt is difficult to be precipitated and the temporal clogging of the flow channel can be effectively suppressed.

According to the production method according to the embodiment of the present invention, in the total solid content of the reaction solution (which is in a state of not being subjected to purification treatment or the like) immediately after the flow type reaction, the content of the by-product via the quaternary salt, which is generated by the reaction between the tertiary amine and the phosgene described above, can be set to be less than 10% by mass, less than 8% by mass, or less than 5% by mass.

The present invention has been described together with the preferred embodiments thereof; however, the present invention is not limited to the above embodiments except for the matters specified in the present invention.

Regarding the above-described embodiment, in the production method according to the embodiment of the present invention, a flow type reaction system can be widely used where the flow type reaction system is
a flow type reaction system of producing a carbonyl compound, including:
a first flow channel into which a triphosgene solution is introduced; a second flow channel into which a tertiary amine solution is introduced; a third flow channel into which an active hydrogen-containing compound solution is introduced; a first joining part at which the first flow channel and the second flow channel are joined; a fourth flow channel which is connected downstream of the first joining part; a second joining part at which the fourth flow channel and the third flow channel are joined; and a reaction pipe which is connected downstream of the second joining part,
in which a solvent of each of the solutions is a non-aqueous organic solvent, and the tertiary amine has a cyclic structure.

In addition, in the production method according to the embodiment of the present invention, a flow type reaction system can be widely used where the flow type reaction system is a flow type reaction system of producing a carbonyl compound, including:
a first flow channel into which a triphosgene solution is introduced; a second flow channel into which a tertiary amine solution is introduced; a third flow channel into which an active hydrogen-containing compound solution is introduced; a joining part at which the first flow channel, the second flow channel, and the third flow channel are joined; and a reaction pipe which is connected downstream of the joining part,
in which a solvent of each of the solutions is a non-aqueous organic solvent, and the tertiary amine has a cyclic structure.

The present invention will be described in more detail based on Examples; however, the present invention is not limited to these Examples.

### Examples

### [Example 1]

An isocyanate compound was synthesized using the flow type reaction system 100 having the constitution illustrated in Fig. 1. The reaction scheme of this synthesis reaction is shown below. In the scheme below, "Ph" is phenyl, and NR₃ is a tertiary amine.

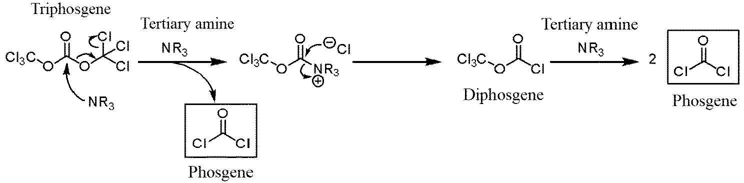

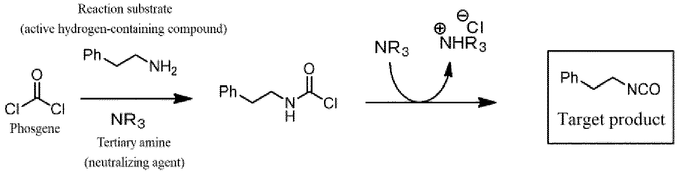

The specific reaction conditions are as follows.

### Liquid feeding pump (not illustrated in the drawing):

All liquid feeding pumps used were PU716B and PU718 manufactured by GL Sciences Inc., and a pulse damper HPD-1, a back pressure valve (44-2361-24) manufactured by NIHON TESCON Co., Ltd., and a relief valve RHA (4 MPa) manufactured by IBS COMPANY were sequentially installed on the side of the flow outlet port.

### Temperature control:

All of the flow channels (I) to (V) and the joining parts M1 and M2 were immersed in water set at 10°C.

### Flow channels (I) to (V):

All flow channels used were a SUS316 tube having an outer diameter of 1.6 mm (1/16 inch) and an inner diameter of 1.0 mm. The length of each of the flow channels is as follows.
Flow channel (I): 0.5 m
Flow channel (II): 0.5 m
Flow channel (III): 0.5 m
Flow channel (IV): 1.0 m
Flow channel (V): 1.0 m

### Joining part (M1, M2) (a T-shaped connector):

SUS T-type mixers having an inner diameter of 0.5 mm, manufactured by IDEX CORPORATION, were used as two T-shaped connectors (M1, M2).

### Triphosgene solution:

A triphosgene solution (triphosgene concentration: 0.0661 M) obtained by dissolving triphosgene in methylene chloride was prepared.

### Tertiary amine solution:

An N-(2-ethylhexyl)morpholine solution (N-(2-ethylhexyl)morpholine concentration: 0.529M) obtained by dissolving N-(2-ethylhexyl)morpholine in methylene chloride was prepared.

### Active hydrogen-containing compound solution:

A phenethylamine solution (phenethylamine concentration: 0.132 M) obtained by dissolving phenethylamine in methylene chloride was prepared.

### Liquid feeding conditions:

Triphosgene solution: 1.0 mL/min
Tertiary amine solution: 1.0 mL/min
Active hydrogen-containing compound solution: 1.0 mL/min

The water content of the solvent of each of the above solutions is shown in the table below.

### Purity of reaction product (isocyanate compound):

A reaction solution was collected from the outlet port (most downstream) of the flow channel (V), diluted 500-fold with a reaction solvent (methylene chloride in Example 1), and the diluted sample was analyzed by gas chromatography under the following conditions to measure the purity. The results are shown in the table below. In the table below, ">97" means that the purity is more than 97% by mass, and "<10" means that the purity is less than 10% by mass.

### - Analysis conditions -

Measuring equipment: GC-3200 (manufactured by GL Sciences Inc.)
Column: APS-1,000 (Teflon, 3φ × 6m, manufactured by GL Sciences Inc.)
Column temperature: 250°C
Carrier gas: Hydrogen (hydrogen gas generator: HG260B, manufactured by GL Sciences Inc.)
Injection volume: 1 µL

The results are shown in the table below.

In the column of impurity in the table below, the proportion (% by mass) of the by-product via the quaternary salt, which is generated by the reaction between the tertiary amine and the phosgene, is shown as "Impurity (% by mass)". This proportion is a proportion of the by-product to the total solid content in the reaction solution after the reaction.

### Evaluation of flow channel clogging:

A pressure gauge was installed in the middle of the flow channel between the tertiary amine solution introduction port (iB) and the joining part (M2) (that is, inside the flow channel (II)), and the pressure after 1 hour passed at the time when the liquid feeding became stable and the reaction was in a steady state, was evaluated as evaluation "A" in a case of less than 0.05 MPa, as evaluation "B" in a case of 0.05 MPa or more and less than 0.1 MPa, and as evaluation "C" in a case of 0.1 MPa or more. The results are shown in the table below.

### [Examples 2 to 32 and Comparative Examples 1 to 5]

Flow type reactions were carried out in the same manner as in Example 1 except that the kinds of flow type reaction systems (the system illustrated in Fig. 1 or 2), solvents, and tertiary amines (the matching between Nos. 1 to 28 and the chemical structures of the tertiary amines are as described above, and in the table below, a tertiary amine "29" is tri-n-butylamine, and a tertiary amine "30" is N,N-diisopropylethylamine) were as shown in the table below. The results are shown in the table below.

In the flow type reaction systems (200) of Fig. 2, a SUS316 tube having an outer diameter of 1.6 mm (1/16 inch) and an inner diameter of 1.0 mm was used as the flow channels (I) to (III) and (V).

In the flow type reaction system (200) of Fig. 2, all the lengths of the flow channels (I) to (III) were set to 0.5 m, and the length of the flow channels (V) was set to 1.0 m. In addition, a SUS cross mixer having an inner diameter of 0.5 mm, manufactured by IDEX CORPORATION, was used as the cross-shaped connector that constitutes the joining part (M1).

**[Table 1]**

| | Tertiary amine | Solvent | Solvent water content (ppm) | Flow type reaction system | Evaluation result | | |
|---|---|---|---|---|---|---|---|
| | | | | | Purity (% by mass) | Impurity (% by mass) | Clogging property |
| Example 1 | 1 | Methylene chloride | <50 | Fig. 1 | >97 | n.d. | A |
| Example 2 | 1 | Methylene chloride | 320 | Fig. 1 | >97 | n.d. | A |
| Example 3 | 1 | Methylene chloride | 910 | Fig. 1 | 95 | n.d. | A |
| Example 4 | 1 | Methylene chloride | 1350 | Fig. 1 | 76 | 2 | A |
| Example 5 | 1 | Toluene | 320 | Fig. 1 | >97 | n.d. | A |
| Example 6 | 2 | Toluene | 320 | Fig. 1 | 94 | n.d. | A |
| Example 7 | 3 | Mesitylene | 360 | Fig. 1 | 79 | 1 | A |
| Example 8 | 4 | Toluene | 320 | Fig. 1 | 90 | n.d. | A |
| Example 9 | 5 | Toluene | 480 | Fig. 1 | 86 | n.d. | A |
| Example 10 | 6 | Methylene chloride | 320 | Fig. 1 | 90 | n.d. | A |
| Example 11 | 7 | Toluene | 320 | Fig. 1 | >97 | n.d. | A |
| Example 12 | 8 | Acetonitrile | 320 | Fig. 1 | 93 | n.d. | A |
| Example 13 | 9 | Toluene | 320 | Fig. 1 | 73 | 3 | B |
| Example 14 | 10 | Methylene chloride | 360 | Fig. 1 | 92 | n.d. | A |
| Example 15 | 11 | Chlorobenzene | 320 | Fig. 2 | 73 | 3 | A |
| Example 16 | 12 | Toluene | 360 | Fig. 1 | 88 | n.d. | A |
| Example 17 | 13 | Xylene | 320 | Fig. 1 | 79 | 2 | A |
| Example 18 | 14 | o-dichlorobenzene | 360 | Fig. 1 | 80 | 1 | A |
| Example 19 | 15 | Tetrahydrofuran | 320 | Fig. 1 | 77 | 5 | A |
| Example 20 | 16 | Methylene chloride | 320 | Fig. 1 | 72 | 4 | B |
| Example 21 | 17 | o-dichlorobenzene | 320 | Fig. 2 | 70 | 4 | A |
| Example 22 | 18 | Chlorobenzene | 360 | Fig. 1 | 81 | 1 | A |
| Example 23 | 19 | Methylene chloride | 320 | Fig. 1 | 71 | 4 | A |
| Example 24 | 20 | Methylene chloride | 480 | Fig. 1 | 68 | 6 | B |
| Example 25 | 21 | Methylene chloride | 320 | Fig. 1 | 73 | 4 | B |
| Example 26 | 22 | Xylene | 360 | Fig. 1 | 73 | 3 | A |
| Example 27 | 23 | Toluene | 320 | Fig. 1 | 75 | 4 | A |
| Example 28 | 24 | Toluene | 320 | Fig. 1 | 60 | 8 | B |
| Example 29 | 25 | Tetrahydrofuran | 320 | Fig. 1 | 79 | 3 | A |
| Example 30 | 26 | Chlorobenzene | 320 | Fig. 1 | 79 | 7 | A |
| Example 31 | 27 | Acetonitrile | 320 | Fig. 1 | 70 | 8 | A |
| Example 32 | 28 | Xylene | 360 | Fig. 1 | 71 | n.d. | A |
| Comparative Example 1 | 1 | Tetrahydrofuran/water=9/1 (mass ratio) | 100000 | Fig. 1 | <10 | 10 | B |
| Comparative Example 2 | 29 | Methylene chloride | 320 | Fig. 1 | 54 | 17 | A |
| Comparative Example 3 | 29 | Methylene chloride | 480 | Fig. 2 | 44 | 28 | B |
| Comparative Example 4 | 30 | Methylene chloride | 360 | Fig. 1 | 53 | 23 | A |
| Comparative Example 5 | 30 | Methylene chloride | 320 | Fig. 2 | <10 | 31 | C |

| | | | | | | | |
|---|---|---|---|---|---|---|---|
| n.d.: undetectable | | | | | | | |

As shown in Table 1 above, in a case where an organic solvent having a large amount of water content was used as the reaction solvent, the purity of the target reaction product was significantly reduced, and the amount of by-product and the amount product via the quaternary salt was also large (Comparative Example 1). In addition, even in a case where a tertiary amine having only a chain-like substituent without having a cyclic structure was used, the purity of the target reaction product was less than 55% by mass, and conversely, the amount of by-product via the quaternary salt increased (Comparative Examples 2 to 5).

On the other hand, in a case where the flow type reaction according to the embodiment of the present invention was applied, it was found that the purity of the target reaction product can be remarkably increased and the generation of by-product via the quaternary salt can be effectively suppressed (Examples 1 to 32). Further, in Examples 1 to 32, the clogging of the flow channel could be suppressed. That is, it can be seen that the tertiary amine specified in the present invention sufficiently functions as a catalyst for converting triphosgene into phosgene and has excellent properties as a highly soluble neutralizing agent in an organic solvent.

### Evaluation of temporal clogging of flow channel:

### [Examples 33 to 48]

Using the flow type reaction system illustrated in Fig. 1, flow type reactions were carried out in the same manner as in Example 1, where the active hydrogen-containing compounds used were set to primary amines shown in the table below. The reaction was carried out continuously for 1 hour, during which the purity of the reaction product, the amount of by-product (impurities), and the occurrence of temporal clogging were examined. The evaluation standards for clogging were the same as above. The results are shown in the table below.

**[Table 2]**

| | Primary amine | Solvent | Tertiary amine | Solvent water content (ppm) | Flow type reaction system | Evaluation result | | | |
|---|---|---|---|---|---|---|---|---|---|
| | | | | | | Product | Purity (% by mass) | Impurity (% by mass) | Clogging property |
| Example 33 | 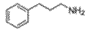 | 1 | Methylene chloride | <50 | Fig. 1 | 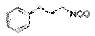 | 94 | n.d. | A |
| Example 34 | 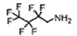 | 1 | Methylene chloride | <50 | Fig. 1 | 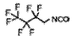 | 89 | n.d. | A |
| Example 35 | 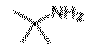 | 1 | Methylene chloride | <50 | Fig. 1 | 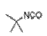 | 80 | 2 | A |
| Example 36 | 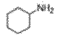 | 1 | Methylene chloride | 350 | Fig. 1 | 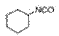 | 79 | n.d. | A |
| Example 37 | 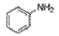 | 7 | Methylene chloride | <50 | Fig. 1 | 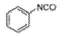 | 85 | n.d. | A |
| Example 38 | 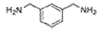 | 7 | Toluene | 120 | Fig. 1 | 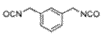 | 83 | 2 | A |
| Example 39 | 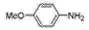 | 1 | Methylene chloride | <50 | Fig. 1 | 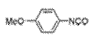 | 92 | n.d. | A |
| Example 40 | 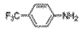 | 1 | Methylene chloride | 200 | Fig. 1 | 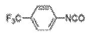 | 91 | <1 | A |
| Example 41 | 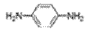 | 1 | Methylene chloride | <50 | Fig. 1 | 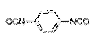 | 84 | n.d. | A |
| Example 42 | 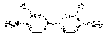 | 2 | Methylene chloride | <50 | Fig, 1 | 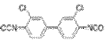 | 71 | 4 | A |
| Example 43 | 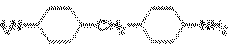 | 7 | Chlorobenzene | <50 | Fig. 1 | 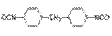 | 78 | <1 | 8 |
| Example 44 | 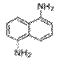 | 7 | o-dichforobenzene | <50 | Fig. 1 | 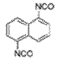 | 70 | <1 | B |
| Example 45 | 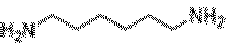 | 7 | Methylene chloride | <50 | Fig. 1 | 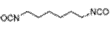 | 79 | n.d. | A |
| Example 46 | 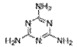 | 2 | Methylene chloride o-dichlorobenzene = 1/1 (mass ratio) | <50 | Fig. 1 | 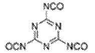 | 72 | 3 | B |
| Example 47 | 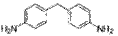 | 7 | o-dichiprobensene | <50 | Fig. 1 | 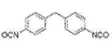 | 80 | n.d. | B |
| Example | 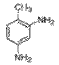 | 1 | Methylene chloride | <50 | Fig. 1 | 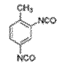 | 67 | 9 | A |

### [Examples 49 to 52]

Using the flow type reaction system illustrated in Fig. 1, flow type reactions were carried out in the same manner as in Example 1, where the active hydrogen-containing compounds used were set to alcohols shown in the table below, and carbonate compounds were obtained. The reaction was carried out continuously for 1 hour, during which the purity of the reaction product, the amount of by-product (impurities), and the occurrence of temporal clogging were examined. The evaluation standards for clogging were the same as above. The results are shown in the table below.

**[Table 3]**

| | Alcohol | Tertiary amine | Solvent | Solvent water content (ppm) | Flow type reaction system | Evaluation result | | | |
|---|---|---|---|---|---|---|---|---|---|
| | | | | | | Product | Purity (% by mass) | Tmpurity (% by mass) | Clogging property |
| Example 49 | 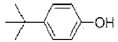 | 1 | Methylene chloride | 250 | Fig. 1 | 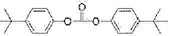 | 78 | 4 | A |
| Example 50 | 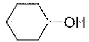 | 1 | Methylene chloride | 250 | Fig. 1 | 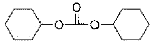 | 80 | 2 | A |
| Example 51 | 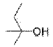 | 1 | Methylene chloride | <5 | Fig. 1 | 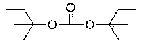 | 68 | 3 | A |
| Example 52 | 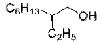 | 1 | Methylene chloride | 250 | Fig. 1 | 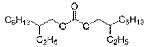 | 71 | n.d. | A |

### [Examples 53 to 59]

Using the flow type reaction system illustrated in Fig. 1, flow type reactions were carried out in the same manner as in Example 1, where the active hydrogen-containing compounds used were set to secondary amines shown in the table below, and carbamoyl chloride was obtained. The reaction was carried out continuously for 1 hour, during which the purity of the reaction product, the amount of by-product (impurities), and the occurrence of temporal clogging were examined. The evaluation standards for clogging were the same as above. The results are shown in the table below.

**[Table 4]**

| | Secondary amine | Tertiary amine | Solvent | Solvent water content (ppm) | Flow type reaction system | Evaluation result | | | |
|---|---|---|---|---|---|---|---|---|---|
| | | | | | | Product | Purity (% by mass) | Impurity (% by mass) | Clogging property |
| Example 53 | 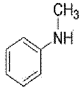 | 1 | Methylene chloride | <5 | Fig. 1 | 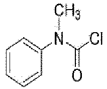 | 92 | 4 | A |
| Example 54 | 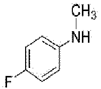 | 1 | Methylene chloride | <5 | Fig. 1 | 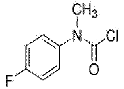 | 85 | 2 | A |
| Example 55 | 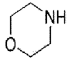 | 1 | Methylene chloride | 150 | Fig. 1 | 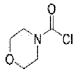 | 9 | 3 | A |
| Example 56 | 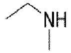 | 1 | Methylene chloride | <5 | Fig. 1 | 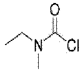 | 90 | n.d. | B |
| Example 57 | 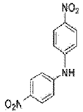 | 1 | Methylene chloride | <5 | Fig. 1 | 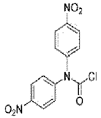 | 82 | 2 | A |
| Example 58 | 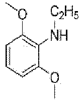 | 1 | Methylene chloride | <5 | Fig. 1 | 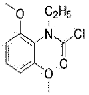 | 88 | 3 | A |
| Example 59 | 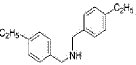 | 1 | Toluene | 300 | Fig. 1 | 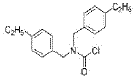 | 81 | 5 | A |

### [Examples 60 to 62]

Using the flow type reaction system illustrated in Fig. 1, flow type reactions were carried out in the same manner as in Example 1, where the active hydrogen-containing compounds used were set to amino acids shown in the table below, and amino acid N-carboxy anhydrides were obtained. The reaction was carried out continuously for 1 hour, during which the purity of the reaction product, the amount of by-product (impurities), and the occurrence of temporal clogging were examined. The evaluation standards for clogging were the same as above. The results are shown in the table below.

**[Table 5]**

| | Amino acid | Tertiary amine | Solvent | Solvent water content (ppm) | Flow type reaction system | Evaluation result | | | |
|---|---|---|---|---|---|---|---|---|---|
| | | | | | | Product | Purity (% by mass) | Impurity (% by mass) | Clogging property |
| Example 60 | 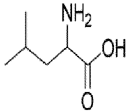 | 1 | Methylene chloride | <5 | Fig. 1 | 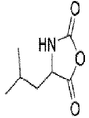 | 95 | 2 | A |
| Example 61 | 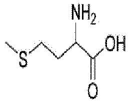 | 1 | Methylene chloride | <5 | Fig. 1 | 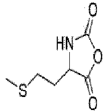 | 94 | n.d. | A |
| Example 62 | 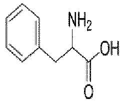 | 1 | Toluene | <5 | Fig. 1 | 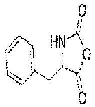 | 89 | 3 | A |

The present invention is based on the new findings that in a case where a series of chemical reactions, in which phosgene is generated from triphosgene, and a carbonyl group is introduced into a reaction substrate by the generated phosgene, are continuously carried out by a flow type reaction, a tertiary amine that function as a conversion catalyst and phosgene easily cause a side reaction, which hinders the higher purity of the target reaction product. This invention has been completed by solving this problem by applying a tertiary amine having a specific structure.

The present invention has been described together with the embodiments of the present invention. However, the inventors of the present invention do not intend to limit the present invention in any part of the details of the description unless otherwise specified, and it is considered that the present invention should be broadly construed without departing from the scope of the invention shown in the attached claims.

This application claims priority based on JP2019-152113 filed in Japan on August 22, 2019.

### Explanation of References

100, 200: flow type reaction system
iA: triphosgene solution introduction port
iB: tertiary amine solution introduction port
iC: active hydrogen-containing compound solution introduction port
I: flow channel having introduction port iA
II: flow channel having introduction port iB
III: flow channel having introduction port iC
IV: reaction flow channel (conversion of triphosgene into phosgene)
V: reaction flow channel (carbonyl group introduction reaction)
M1, M2; joining part

## Claims

1. A method of producing a carbonyl compound by a flow type reaction, comprising:
introducing a triphosgene solution, a tertiary amine solution, and an active hydrogen-containing compound solution into flow channels different from each other to cause the respective solutions to flow inside the respective flow channels, joining the respective solutions that flow inside the respective flow channels simultaneously or sequentially so that a reaction between phosgene and an active hydrogen-containing compound occurs, and obtaining a carbonyl compound in a joining solution,
wherein a non-aqueous organic solvents is used as a solvent of each of the respective solutions and a compound having a cyclic structure is used as the tertiary amine; wherein a water content of the non-aqueous organic solvent is 1,000 ppm or less.

2. The method of producing a carbonyl compound according to claim 1,
wherein the tertiary amine has at least one group of an alkyl group having 1 to 40 carbon atoms, a cycloalkyl group having 3 to 40 carbon atoms, an alkoxy group having 1 to 40 carbon atoms, or a polyether group having 2 to 40 carbon atoms.

3. The method of producing a carbonyl compound according to claim 1 or 2,
wherein the tertiary amine has an alkyl group having 2 to 30 carbon atoms.

4. The method of producing a carbonyl compound according to any one of claims 1 to 3,
wherein the tertiary amine has a branched alkyl group.

5. The method of producing a carbonyl compound according to any one of claims 1 to 4,
wherein the tertiary amine has an alicyclic structure.

6. The method of producing a carbonyl compound according to any one of claims 1 to 5,
wherein the tertiary amine has a heterocyclic ring structure having a nitrogen atom as a ring-constituting atom.

7. The method of producing a carbonyl compound according to any one of claims 1 to 6,
wherein the tertiary amine has an oxygen atom.

8. The method of producing a carbonyl compound according to any one of claims 1 to 7,
wherein the tertiary amine has a morpholine ring structure.

9. The method of producing a carbonyl compound according to any one of claims 1 to 8,
wherein the tertiary amine has an aromatic heterocyclic ring structure having a nitrogen atom as a ring-constituting atom.

10. The method of producing a carbonyl compound according to any one of claims 1 to 9,
wherein the tertiary amine has a pyridine ring structure.

11. The method of producing a carbonyl compound according to any one of claims 1 to 10,
wherein the triphosgene solution and the tertiary amine solution are joined to generate a phosgene solution, and the phosgene solution and the active hydrogen-containing compound solution are joined to obtain a carbonyl compound in the joining solution.

12. The method of producing a carbonyl compound according to any one of claims 1 to 11,
wherein the active hydrogen-containing compound is at least one of a primary amine, a secondary amine, an alcohol, a thiol, a carboxylic acid, or an amino acid, and the active hydrogen-containing compound is preferably a primary amine.

13. A flow type reaction system of producing a carbonyl compound, comprising:
a first flow channel into which a triphosgene solution is introduced; a second flow channel into which a tertiary amine solution is introduced; a third flow channel into which an active hydrogen-containing compound solution is introduced; a first joining part at which the first flow channel and the second flow channel are joined; a fourth flow channel which is connected downstream of the first joining part; a second joining part at which the fourth flow channel and the third flow channel are joined; and a reaction pipe which is connected downstream of the second joining part,
wherein a solvent of each of the solutions is a non-aqueous organic solvents, and the tertiary amine has a cyclic structure;
wherein a water content of the non-aqueous organic solvent is 1,000 ppm or less.

14. A flow type reaction system of producing a carbonyl compound, comprising:
a first flow channel into which a triphosgene solution is introduced;
a second flow channel into which a tertiary amine solution is introduced;
a third flow channel into which an active hydrogen-containing compound solution is introduced;
a joining part at which the first flow channel, the second flow channel, and the third flow channel are joined; and
a reaction pipe which is connected downstream of the joining part,
wherein a solvent of each of the solutions is a non-aqueous organic solvents, and the tertiary amine has a cyclic structure;
wherein a water content of the non-aqueous organic solvent is 1,000 ppm or less.

## Patentansprüche

1. Verfahren des Produzierens einer Carbonylverbindung durch eine Reaktion des Strömungstyps, umfassend:
Einführen einer Triphosgenlösung, einer Lösung von tertiärem Amin und einer Lösung von aktivwasserstoffhaltiger Verbindung in voneinander verschiedene Strömungskanäle, um zu bewirken, dass die jeweiligen Lösungen innerhalb der jeweiligen Strömungskanäle strömen;
Zusammenführen der jeweiligen Lösungen, die innerhalb der jeweiligen Strömungskanäle gleichzeitig oder nacheinander strömen, so dass eine Reaktion zwischen Phosgen und einer aktivwasserstoffhaltigen Verbindung auftritt; und
Erhalten einer Carbonylverbindung in einer Zusammenführungslösung,
wobei ein nicht wässriges organisches Lösungsmittel als ein Lösungsmittel jeder der jeweiligen Lösungen verwendet wird und eine Verbindung mit einer zyklischen Struktur als das tertiäre Amin verwendet wird, wobei ein Wassergehalt des nicht wässrigen organischen Lösungsmittels 1.000 ppm oder weniger beträgt.

2. Verfahren des Produzierens einer Carbonylverbindung nach Anspruch 1,
wobei das tertiäre Amin mindestens eine Gruppe einer Alkylgruppe mit 1 bis 40 Kohlenstoffatomen, einer Cycloalkylgruppe mit 3 bis 40 Kohlenstoffatomen, einer Alkoxygruppe mit 1 bis 40 Kohlenstoffatomen oder einer Polyethergruppe mit 2 bis 40 Kohlenstoffatomen aufweist.

3. Verfahren des Produzierens einer Carbonylverbindung nach Anspruch 1 oder 2,
wobei das tertiäre Amin eine Alkylgruppe mit 2 bis 30 Kohlenstoffatomen aufweist.

4. Verfahren des Produzierens einer Carbonylverbindung nach einem der Ansprüche 1 bis 3,
wobei das tertiäre Amin eine verzweigte Alkylgruppe aufweist.

5. Verfahren des Produzierens einer Carbonylverbindung nach einem der Ansprüche 1 bis 4,
wobei das tertiäre Amin eine alizyklische Struktur aufweist.

6. Verfahren des Produzierens einer Carbonylverbindung nach einem der Ansprüche 1 bis 5,
wobei das tertiäre Amin eine heterozyklische Ringstruktur, die ein Stickstoffatom als ein ringbildendes Atom aufweist, aufweist.

7. Verfahren des Produzierens einer Carbonylverbindung nach einem der Ansprüche 1 bis 6,
wobei das tertiäre Amin ein Sauerstoffatom aufweist.

8. Verfahren des Produzierens einer Carbonylverbindung nach einem der Ansprüche 1 bis 7,
wobei das tertiäre Amin eine Morpholin-Ringstruktur aufweist.

9. Verfahren des Produzierens einer Carbonylverbindung nach einem der Ansprüche 1 bis 8,
wobei das tertiäre Amin eine aromatische heterozyklische Ringstruktur, die ein Stickstoffatom als ein ringbildendes Atom aufweist, aufweist.

10. Verfahren des Produzierens einer Carbonylverbindung nach einem der Ansprüche 1 bis 9,
wobei das tertiäre Amin eine Pyridin-Ringstruktur aufweist.

11. Verfahren des Produzierens einer Carbonylverbindung nach einem der Ansprüche 1 bis 10,
wobei die Triphosgenlösung und die Lösung von tertiärem Amin zusammengeführt werden, um eine Phosgenlösung zu erzeugen, und die Phosgenlösung und die Lösung von aktivwasserstoffhaltiger Verbindung zusammengeführt werden, um eine Carbonylverbindung in der Zusammenführungslösung zu erhalten.

12. Verfahren des Produzierens einer Carbonylverbindung nach einem der Ansprüche 1 bis 11,
wobei die aktivwasserstoffhaltige Verbindung mindestens eines von einem primären Amin, einem sekundären Amin, einem Alkohol, einem Thiol, einer Carbonsäure und einer Aminosäure ist und die aktivwasserstoffhaltige Verbindung bevorzugt ein primäres Amin ist.

13. Reaktionssystem des Strömungstyps des Produzierens einer Carbonylverbindung, umfassend:
einen ersten Strömungskanal, in den eine Triphosgenlösung eingeführt wird;
einen zweiten Strömungskanal, in den eine Lösung von tertiärem Amin eingeführt wird;
einen dritten Strömungskanal, in den eine Lösung von aktivwasserstoffhaltiger Verbindung eingeführt wird;
einen ersten Verbindungsteil, an dem der erste Strömungskanal und der zweite Strömungskanal zusammengeführt werden;
einen vierten Strömungskanal, der stromabwärts des ersten Verbindungsteils verbunden ist;
einen zweiten Verbindungsteil, an dem der vierte Strömungskanal und der dritte Strömungskanal zusammengeführt werden; und
ein Reaktionsrohr, das stromabwärts des zweiten Verbindungsteils verbunden ist,
wobei ein Lösungsmittel jeder der Lösungen ein nicht wässriges organisches Lösungsmittel ist und das tertiäre Amin eine zyklische Struktur aufweist, wobei ein Wassergehalt des nicht wässrigen organischen Lösungsmittels 1.000 ppm oder weniger beträgt.

14. Reaktionssystem des Strömungstyps des Produzierens einer Carbonylverbindung, umfassend:
einen ersten Strömungskanal, in den eine Triphosgenlösung eingeführt wird;
einen zweiten Strömungskanal, in den eine Lösung von tertiärem Amin eingeführt wird;
einen dritten Strömungskanal, in den eine Lösung von aktivwasserstoffhaltiger Verbindung eingeführt wird;
einen Verbindungsteil, an dem der erste Strömungskanal, der zweite Strömungskanal und der dritte Strömungskanal zusammengeführt werden; und
ein Reaktionsrohr, das stromabwärts des Verbindungsteils verbunden ist,
wobei ein Lösungsmittel jeder der Lösungen ein nicht wässriges organisches Lösungsmittel ist und das tertiäre Amin eine zyklische Struktur aufweist, wobei ein Wassergehalt des nicht wässrigen organischen Lösungsmittels 1.000 ppm oder weniger beträgt.

## Revendications

1. Procédé de production d'un composé carbonylé par une réaction en flux, comprenant :
introduire une solution de triphosgène, une solution d'amine tertiaire et une solution de composé contenant de l'hydrogène actif dans des canaux d'écoulement différents les uns des autres pour amener les solutions respectives à s'écouler à l'intérieur des canaux d'écoulement respectifs ;
joindre les solutions respectives qui s'écoulent à l'intérieur des canaux d'écoulement respectifs simultanément ou séquentiellement de sorte qu'une réaction entre du phosgène et un composé contenant de l'hydrogène actif a lieu ; et
obtenir un composé carbonyle dans une solution de jonction,
dans lequel un solvant organique non aqueux est utilisé comme un solvant de chacune des solutions respectives et un composé ayant une structure cyclique est utilisé comme l'amine tertiaire, dans lequel une teneur en eau du solvant organique non aqueux est de 1 000 ppm ou moins.

2. Procédé de production d'un composé carbonylé selon la revendication 1,
dans lequel l'amine tertiaire a au moins un groupe d'un groupe alkyle ayant de 1 à 40 atomes de carbone, d'un groupe cycloalkyle ayant de 3 à 40 atomes de carbone, d'un groupe alkoxy ayant de 1 à 40 atomes de carbone ou d'un groupe polyéther ayant de 2 à 40 atomes de carbone.

3. Procédé de production d'un composé carbonylé selon la revendication 1 ou la revendication 2,
dans lequel l'amine tertiaire a un groupe alkyle ayant de 2 à 30 atomes de carbone.

4. Procédé de production d'un composé carbonylé selon l'une quelconque des revendications 1 à 3,
dans lequel l'amine tertiaire a un groupe alkyle ramifié.

5. Procédé de production d'un composé carbonylé selon l'une quelconque des revendications 1 à 4,
dans lequel l'amine tertiaire a une structure alicyclique.

6. Procédé de production d'un composé carbonylé selon l'une quelconque des revendications 1 à 5,
dans lequel l'amine tertiaire a une structure de cycle hétérocyclique ayant un atome d'azote comme un atome constitutif de cycle.

7. Procédé de production d'un composé carbonylé selon l'une quelconque des revendications 1 à 6,
dans lequel l'amine tertiaire a un atome d'oxygène.

8. Procédé de production d'un composé carbonylé selon l'une quelconque des revendications 1 à 7,
dans lequel l'amine tertiaire a une structure de cycle morpholine.

9. Procédé de production d'un composé carbonylé selon l'une quelconque des revendications 1 à 8,
dans lequel l'amine tertiaire a une structure de cycle hétérocyclique aromatique ayant un atome d'azote comme un atome constitutif de cycle.

10. Procédé de production d'un composé carbonylé selon l'une quelconque des revendications 1 à 9,
dans lequel l'amine tertiaire a une structure de cycle pyridine.

11. Procédé de production d'un composé carbonylé selon l'une quelconque des revendications 1 à 10,
dans lequel la solution de triphosgène et la solution d'amine tertiaire sont jointes pour générer une solution de phosgène, et la solution de phosgène et la solution de composé contenant de l'hydrogène actif sont jointes pour obtenir un composé carbonyle dans la solution de jonction.

12. Procédé de production d'un composé carbonylé selon l'une quelconque des revendications 1 à 11,
dans lequel le composé contenant de l'hydrogène actif est au moins l'une d'une amine primaire, d'une amine secondaire, d'un alcool, d'un thiol, d'un acide carboxylique ou d'un acide aminé, et le composé contenant de l'hydrogène actif est de préférence une amine primaire.

13. Système de réaction en flux pour produire un composé carbonylé, comprenant :
un premier canal d'écoulement dans lequel une solution de triphosgène est introduite ;
un deuxième canal d'écoulement dans lequel une solution d'amine tertiaire est introduite ;
un troisième canal d'écoulement dans lequel une solution de composé contenant de l'hydrogène actif est introduite ;
une première partie de jonction à laquelle le premier canal d'écoulement et le deuxième canal d'écoulement sont joints ;
un quatrième canal d'écoulement qui est connecté en aval de la première partie de jonction ;
une deuxième partie de jonction à laquelle le quatrième canal d'écoulement et le troisième canal d'écoulement sont joints ; et
un tuyau de réaction qui est connecté en aval de la deuxième partie de jonction,
dans lequel un solvant de chacune des solutions est un solvant organique non aqueux, et l'amine tertiaire a une structure cyclique, dans lequel une teneur en eau du solvant organique non aqueux est de 1 000 ppm ou moins.

14. Système de réaction en flux pour produire un composé carbonylé, comprenant :
un premier canal d'écoulement dans lequel une solution de triphosgène est introduite ;
un deuxième canal d'écoulement dans lequel une solution d'amine tertiaire est introduite ;
un troisième canal d'écoulement dans lequel une solution de composé contenant de l'hydrogène actif est introduite ;
une partie de jonction à laquelle le premier canal d'écoulement, le deuxième canal d'écoulement et le troisième canal d'écoulement sont joints ; et
un tuyau de réaction qui est connecté en aval de la partie de jonction,
dans lequel un solvant de chacune des solutions est un solvant organique non aqueux, et l'amine tertiaire a une structure cyclique, dans lequel une teneur en eau du solvant organique non aqueux est de 1 000 ppm ou moins.
